# FASCICULE DE BREVET EUROPEEN

(11) **EP 2 131 873 B1**
(45) Date de publication et mention de la délivrance du brevet: **22.02.2012**
(21) Numéro de dépôt: 08775678.9
(22) Date de dépôt: 05.03.2008
(51) Int. Cl.: A61L 2/22, A61L 2/24, A61L 9/14, F24F 3/16, B05B 17/06

(54) **PROCEDE DE DESINFECTION DE SURFACE PAR VOIE AERIENNE**
VERFAHREN ZUR DESINFEKTION EINER OBERFLÄCHE MITTELS LUFTWEG
METHOD FOR DISINFECTING A SURFACE BY AERIAL WAY

(30) Priorité: 05.03.2007 FR 0753650
(43) Date de publication de la demande: 16.12.2009
(73) Titulaire: ARECO FINANCES ET TECHNOLOGIE - ARFITEC, 06130 Grasse (FR)
(72) Inventeur: CORVISIER, Gérard, F-06220 Vallauris (FR); GUERRIN, Fabien, F-06250 Mougins (FR)
(74) Mandataire: Remy, Vincent Noel Paul
(86) Numéro de dépôt international: PCT/FR2008/050375
(87) Numéro de publication internationale: WO 2008/125776

(56) Documents cités:
- EP-A- 1 611 905
- WO-A-00/74734
- WO-A-01/70282
- AT-B- 391 934
- FR-A- 2 721 839
- GB-A- 2 354 443
- US-A- 4 512 951

## Description

La présente invention concerne le domaine de la désinfection de surface par voie aérienne, par diffusion de biocide liquide dans l'air.

Elle s'applique notamment à la désinfection de salles propres, par exemple :
- des blocs opératoires en milieu hospitalier,
- des laboratoires pharmaceutiques, en particulier de leurs salles de fabrication et de conditionnement,
- des laboratoires manipulant des micro organismes pathogènes, ou nécessitant la destruction de tels micro organismes pathogènes,
- des salles blanches de l'industrie de microélectronique,
- des salles propres de l'industrie agroalimentaire,
- des locaux des animaleries et de certains secteurs de l'élevage, par exemple les écloseries.

Dans ces locaux, des désinfections ou des décontaminations sont régulièrement effectuées, d'abord par lavage des sols et des murs au moyen de désinfectants de surface (javel, ammoniums quaternaires, cocktail de désinfectants), puis par voie aérienne (DSVA) pour compléter la première étape et parfaire l'opération.

On parvient ainsi à éliminer les bactéries, les virus, les spores et les moisissures, dans des proportions importantes. En effet, on parle de désinfection en cas de réduction de la concentration de ces micro organismes d'un facteur 1000 (c'est-à-dire que s'il existait 1 million des micro organismes ciblés par mètre cube (*m*³), après désinfection il n'en restera que 1000), ce niveau étant désigné « log3 », et on parle de décontamination en cas de réduction de la concentration d'un facteur 1 000 000 (en reprenant le même exemple, il ne restera que 1 micro organisme ciblé par m3), ce niveau étant désigné « log6 ».

Du document WO 0 170 282, on connaît un procédé de désinfection par voie aérienne d'une enceinte fermée consistant à :
- abaisser l'humidité relative dans l'enceinte à un niveau assez bas, de manière que les micro organismes présents sur les parois de l'enceinte soient secs,
- faire circuler un gaz porteur dans l'enceinte, à une température supérieure à la température ambiante,
- introduire un biocide dans le gaz porteur, en quantité suffisante pour saturer ce dernier de manière que le refroidissement du gaz dans l'enceinte provoque la condensation du biocide sur les parois de celle-ci,
- maintenir les conditions favorables au maintien du condensât de biocide sur les parois pendant un temps suffisant pour détruire les micro organismes,
- rincer l'enceinte en y faisant circuler de l'air neuf.

Ce procédé semble fournir de bons résultats, mais il présente notamment l'inconvénient que les étapes de préparation de l'enceinte, avant introduction du biocide, sont contraignantes et consommatrices de temps et d'énergie.

La présente invention vise à fournir un procédé qui, tout en fournissant des résultats aussi bons, voire meilleurs, ne présente pas cet inconvénient.

La présente invention a pour objet un procédé selon la revendication 1.

De préférence la taille des micro gouttelettes est très petite et ces micro gouttelettes sont calibrées (c'est-à-dire qu'il existe une homogénéité de taille entre toutes les micro gouttelettes). Bien que cela dépende de l'hygrométrie du gaz porteur, une micro gouttelette de 2 micromètres passe à une taille inférieure à 0,1 micromètre en moins d'une seconde, même en hygrométrie quasi saturée. En outre, la surface totale de contact de telles micro gouttelettes est très supérieure, en général 2,5 fois supérieure, à celle de gouttes de plus de 5 micromètres.

Ainsi, grâce à l'invention, le biocide liquide passe à un état assimilable à un gaz et se diffuse partout dans l'enceinte.

Ce procédé présente notamment l'avantage que le biocide peut être injecté de façon massive dans une enceinte ou un ensemble d'enceintes à traiter, ce qui, pour la plupart des biocides, provoque un effet choc renforçant son efficacité. On obtient par exemple de très bons résultats en injectant environ 3 kg de biocides dans l'enceinte en 15 à 20 minutes dans un ensemble d'enceintes de volume total d'environ 250 mètres cube (*m*³).

Un autre avantage du procédé est que les micro gouttelettes de biocide se micro condensent sur les parois de l'enceinte sans provoquer de suintement, ce qui rend notamment possible l'utilisation des gaines d'aéraulique d'une centrale de traitement d'air (CTA) pour diffuser le biocide. L'invention permet alors d'utiliser en synergie des fonctions de chauffage, de refroidissement, d'assèchement, et de renouvellement d'air, de cette centrale. Par ailleurs, la désinfection de conduits d'aéraulique et de la centrale de traitement d'air sont également assurés.

Grâce à l'invention, la concentration en biocide dans le gaz porteur est accrue du fait que le gaz porteur est chauffé à la température de transport avant que les micro gouttelettes de biocide n'y soient introduites. Les micro gouttelettes injectées, qui se retrouvent dans un gaz porteur plus chaud qu'elles, s'évaporent sous l'effet de ce chauffage, et diminuent très rapidement de taille pour se transformer en vapeur sur une courte distance (généralement inférieure à 1 mètre), ce qui accroît la concentration du biocide et, ce faisant, son efficacité. Si, au contraire, le biocide était chauffé à une température plus élevée que celle du gaz porteur, le risque serait que les micro gouttelettes condensent de l'eau présente dans le gaz porteur, provoquant une augmentation de la taille des gouttelettes, ce qui allongerait la zone d'évaporation. En outre, cette eau condensée diluerait le biocide et réduirait son efficacité. Ceci augmenterait les risques de mouillage des parois de l'enceinte et diminuerait l'efficacité de l'injection.

Ainsi, de préférence, les micro gouttelettes sont générées à température ambiante, c'est-à-dire qu'elles ne sont pas chauffées avant d'être injectées dans le gaz porteur, et qu'elles ont donc une température inférieure à la température de transport.

On notera que, l'évaporation de l'eau et du biocide étant endothermique, l'élévation de température du gaz porteur doit être suffisante pour compenser cet effet endothermique et, en outre, pour élever la température des vapeurs de biocide véhiculées.

Dans un mode de mise en oeuvre particulier de l'invention, la génération de micro gouttelettes est réalisée à l'aide d'un générateur à ultrasons à buse acoustique du type de celui décrit dans la demande de brevet de la demanderesse FR 2 721 839.

Dans un autre mode de mise en oeuvre de l'invention, compatible avec le précédent, lors de l'étape consistant à abaisser la température du gaz porteur circulant dans l'enceinte, on fixe également l'hygrométrie relative dans ce gaz porteur en dessous de 60%, voire si possible en dessous de 50%, l'idéal étant 40%. On améliore ainsi la diminution de taille très rapide des micro gouttelettes, ce qui augmente encore l'efficacité de la désinfection. En outre, avec des très petites gouttelettes, de l'ordre de 0,3 micromètres, le biocide peut traverser des filtres à très haute efficacité.

Dans un mode de mise en oeuvre préféré, on utilise, comme biocide liquide, du peroxyde d'hydrogène (H2O2). Un peroxyde d'hydrogène concentré à 30% dans de l'eau constitue un biocide liquide approprié à la mise en oeuvre de l'invention. Par exemple, d'autres agents pourraient être mélangés au peroxyde d'hydrogène, par exemple des agents mouillants, des ions argents, etc.

Selon d'autres caractéristiques avantageuses de l'invention, qui peuvent être prises seules ou en combinaison :
- Le biocide liquide est de l'acide péracétique. Dans ce cas, les contraintes de température et d'hygrométrie sont moins importantes que dans le cas où le biocide est du peroxyde d'hydrogène.
- Le biocide liquide est un mélange de peroxyde d'hydrogène et d'acide péracétique, comprenant de préférence entre 2% et 5% d'acide péracétique.
- Le gaz porteur est de l'air.
- Une fois la température du gaz porteur égale à la température de refroidissement, on stabilise la température des parois de l'enceinte en maintenant la circulation du gaz porteur pendant une durée allant de 20 à 50 minutes.
- L'injection des micro gouttelettes de biocide liquide dans le gaz porteur porté à la température de transport dure entre 20 et 45 minutes.
- Une fois la quantité souhaitée de biocide injectée dans le gaz porteur, on fait circuler le gaz porteur chargé du biocide dans l'enceinte pendant 30 à 60 minutes.
- Eventuellement, on régule la concentration du biocide dans le gaz porteur pendant cette période de 30 à 60 minutes.
- Une fois le traitement terminé, on rince l'enceinte en y faisant circuler un gaz dépourvu de biocide en brassant le gaz à vitesse maximale, de préférence en le chauffant. La durée de ce rinçage dépend du volume à rincer, du débit de renouvellement de ce volume, et/ou de la présence, dans les enceintes traitées, de matériaux susceptibles d'absorber du peroxyde d'hydrogène et de le relâcher ultérieurement. Par exemple, le rinçage peut être effectué pendant 120 à 300 minutes.

La présente invention a aussi pour objet l'utilisation d'une centrale de traitement de l'air comportant, en plus de ses moyens usuels de chauffage, de refroidissement, d'assèchement, et de renouvellement de l'air, de moyens pour générer des micro gouttelettes d'un biocide, de granulométrie voisine de 2 micromètres, ainsi que des moyens pour injecter ces micro gouttelettes dans l'air circulant dans ladite centrale.

Dans un mode de réalisation particulier, la centrale de traitement d'air, ou un conduit d'aéraulique située en amont ou en aval de cette centrale de traitement d'air, comporte une boucle aéraulique en parallèle des moyens de génération des micro-gouttelettes, comprenant des matériaux destinés à catalyser le peroxyde d'hydrogène pendant la phase de rinçage. Le flux de gaz de rinçage ne passe par cette boucle aéraulique qu'en phase de rinçage.

L'invention a encore pour objet l'utilisation un générateur de micro gouttelettes par ultrasons à buse acoustique, comportant des moyens d'alimentation et de vidange alternées en biocide et en eau pour des phases d'injection de micro gouttelettes de biocide et des phases de nettoyage du générateur.

Dans un mode de réalisation particulier, le générateur comporte une réserve d'un catalyseur pour catalyser le peroxyde d'hydrogène pendant la phase de rinçage.

Dans le but de mieux faire comprendre l'invention, on va en décrire maintenant un exemple de mise en oeuvre en référence aux dessins annexés dans lesquels :
- la figure 1 est une vue d'ensemble d'une centrale de traitement d'air utilisée selon un exemple de mode de réalisation de l'invention,
- la figure 2 est une vue d'ensemble d'un générateur à ultrasons branché sur la centrale de traitement de l'air de la figure 1,
- la figure 3 est une vue en coupe selon II-II du générateur de la figure 2,
- la figure 4 représente les étapes d'un procédé de désinfection selon l'invention.

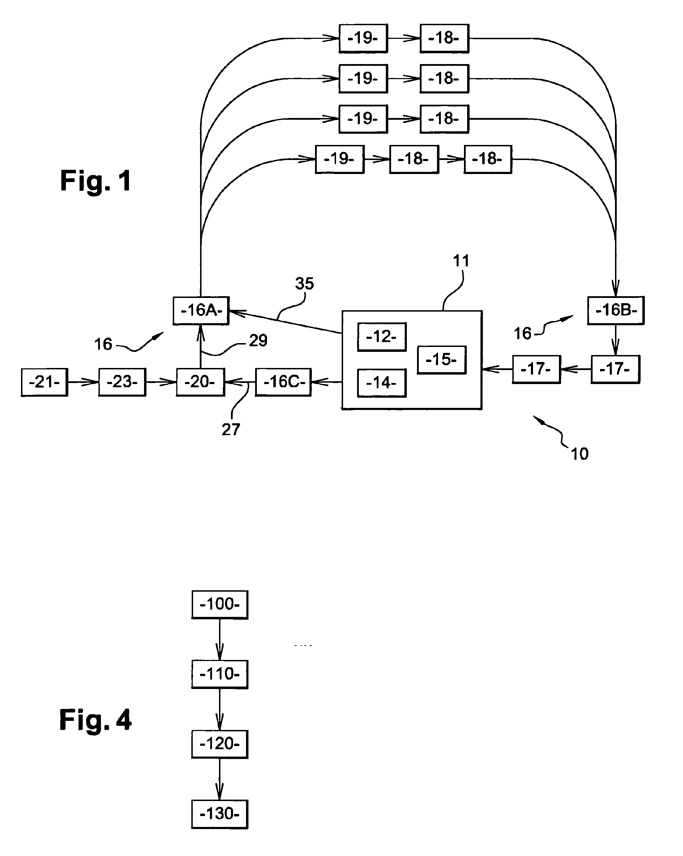

On a représenté sur la figure 1 une centrale de traitement d'air utilisée selon un exemple de mode de réalisation de l'invention. Cette centrale de traitement d'air est désignée par la référence générale 10.

De manière classique, la centrale de traitement de l'air 10 comporte des moyens 17 de filtration, par exemple des rampes de filtration, des moyens classique 11 de climatisation, comprenant des moyens de chauffage 12 et de refroidissement 14, et des moyens 15 d'assèchement de gaz. La centrale 10 comporte en outre des moyens 16 de mise en circulation du gaz et de renouvellement de ce gaz. Généralement, ce gaz mis en circulation est de l'air.

Les moyens 16 de mise en circulation du gaz et de renouvellement d'air comprennent par exemple des ventilateurs de soufflage 16A et de reprise d'air 16B, agencés respectivement en entrée et en sortie d'une enceinte 18, afin d'y faire circuler de l'air traité par la centrale et afin de renouveler cet air. En variante, l'air traité pourrait être mis en circulation dans une pluralité d'enceintes 18 disposées en série ou en dérivation. De préférence, des filtres 19 très haute efficacité sont agencés en amont de chaque enceinte 18.

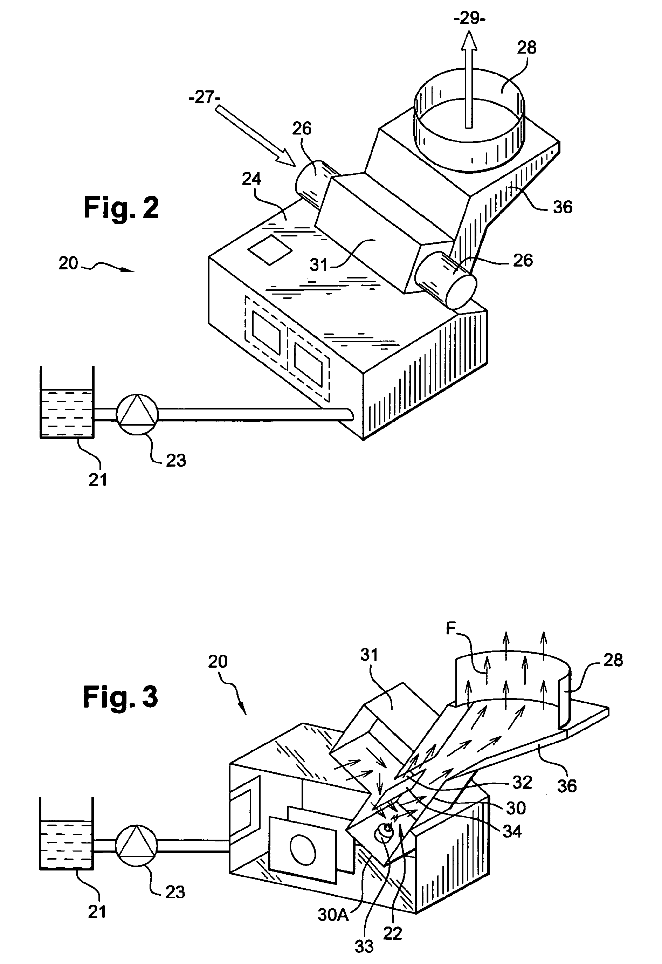

La centrale de traitement d'air comporte en outre un générateur à ultrasons 20, représenté plus en détail sur les figures 2 et 3.

Ce générateur 20 à ultrasons comprend des moyens 22 pour générer des micro gouttelettes d'un biocide, de granulométrie voisine de 2 micromètres, ainsi que des moyens pour injecter ces micro gouttelettes dans l'air circulant dans ladite centrale.

A cet effet, le générateur 20 comporte un boîtier 24 alimenté en courant électrique par un fil conducteur. Un réservoir de biocide 21 et une pompe de dosage 23 asservie au générateur 20 alimentent ce générateur 20 en biocide. Un premier conduit 26 flexible, de diamètre 100 mm environ, relie le générateur 20 à une arrivée d'air 27 de la centrale de traitement de l'air, tandis qu'un autre conduit flexible 28, plus gros, par exemple ayant un diamètre de 250 mm, relie le générateur 20 à au moins un conduit d'aéraulique 29 de départ d'air situé en amont (ou, en variante, en aval) du ventilateur de soufflage 16A. En variante, le conduit aéraulique 29 pourrait aussi être situé en amont ou en aval du ventilateur de reprise 16B. En variante, le générateur 20 pourrait comporter plusieurs conduits 26 d'entrée ou plusieurs conduits 28 de sortie. On notera que le conduit de sortie 28 est de préférence plus gros que le conduit d'entrée 26, de façon à effectuer une détente de l'air, ainsi qu'une réduction de sa vitesse, et donc de réduire les condensations.

Par exemple, l'arrivée d'air 27 est reliée à un ventilateur 16C situé en aval des moyens de chauffage 12 et de refroidissement 14, et des moyens 15 d'assèchement de gaz par rapport au sens de circulation de l'air.

Le départ 29 d'air chargé de micro gouttelettes de biocide est alors relié au ventilateur 16A, en amont de l'enceinte 18, afin de faire circuler dans cette enceinte 18 de l'air comprenant des micro gouttelettes générées par le générateur à ultrasons 20.

Comme on le voit plus particulièrement sur la vue en coupe de la figure 2, l'arrivée d'air 27 se produit latéralement par rapport à un collecteur 31, ici de forme parallélépipédique, qui possède une fente 32 débouchant dans une chambre 30, au-dessus d'une buse acoustique 33 destinée à générer les micro gouttelettes comme cela est connu de FR 2 721 839. Une plaque 34 de déviation d'air est agencée en sortie de la fente 32 afin de dévier l'air à la fois vers la buse acoustique 33, de façon à emporter les micro gouttelettes générées dans ce flux d'air, et vers le conduit de sortie 28 afin d'y transporter ces micro gouttelettes.

On notera que la chambre 30 est conformée pour former en son fond 30A une réserve de biocide liquide dans lequel baigne la buse 33 comme cela est connu.

Les flèches F de la figure 2 représentent la circulation de l'air dans le générateur 20.

Le générateur 20 comporte, en aval de la buse acoustique 33 par rapport au sens de déplacement de l'air, une embouchure de pavillon 36 aboutissant au second conduit flexible 28, de forme évasée de manière à réduire la vitesse de l'air, augmenter le temps d'évaporation et limiter le plus possible les condensations qui pourraient se produire sur les parois internes des conduits et de ladite embouchure 36.

Une centrale de traitement d'air 10 comprenant un tel générateur à ultrasons 20 permet de réaliser un procédé de désinfection de surface par voie aérienne, par diffusion de biocide liquide dans l'air, du type dans lequel on envoie un biocide dans un gaz porteur circulant dans une ou plusieurs enceintes 18, par exemple un sas de désinfection de volume compris entre 10 et 40 *m*³, ou un ensemble d'enceintes distinctes de volume total compris entre 40 et 1000 *m*³. Dans le cas d'un ensemble d'enceintes 18, ces enceintes peuvent être disposées en série ou en parallèle par rapport à la centrale de traitement d'air.

Le procédé de désinfection, dont les étapes sont représentées sur la figure 4, comporte une première étape 100 d'abaissement de la température du gaz, de préférence de l'air, circulant dans l'enceinte jusqu'à une température de refroidissement d'au plus 22°C, de préférence au plus 19°C, par exemple 18°C, jusqu'à ce que les parois de l'enceinte aient atteint cette même température. Cet abaissement de la température de l'air est réalisé grâce aux moyens de refroidissement 14 de la centrale de traitement de l'air.

De préférence, une fois la température du gaz porteur égale à la température de refroidissement, on stabilise la température de parois de l'enceinte 18 en maintenant la circulation du gaz porteur pendant une durée dépendant de l'inertie du local et de son isolation thermique, allant de 20 à 50 minutes.

De préférence également, lors de cette étape 100 d'abaissement de la température de l'air porteur circulant dans l'enceinte, on fixe également l'hygrométrie relative dans ce gaz porteur en dessous de 60%, voire si possible en dessous de 50%, l'idéal étant 40%, à l'aide des moyens 15 d'assèchement de l'air.

Le procédé comporte ensuite une étape 110 de réchauffement de l'air circulant dans l'enceinte jusqu'à une température de transport d'au moins 35°C, de préférence au moins 40°C, par exemple à 41 °C. Cette augmentation de la température de l'air est réalisée grâce aux moyens de chauffage 12 de la centrale de traitement de l'air.

Le procédé comporte également une étape 120 de génération de micro gouttelettes de biocide, de granulométrie voisine de 2 micromètres, et d'injection de ces micro gouttelettes dans l'air porteur circulant dans l'enceinte à la température de transport. Cette étape 120 est réalisée à l'aide du générateur 20 décrit précédemment.

Pour une désinfection efficace, on utilise de préférence, comme biocide liquide, du peroxyde d'hydrogène (H2O2), par exemple une solution de peroxyde d'hydrogène concentré à 30% dans de l'eau. En variante, le biocide liquide pourrait être de l'acide péracétique ou tout autre biocide liquide efficace, par exemple un mélange de peroxyde d'hydrogène et d'acide péracétique, comprenant entre 2% et 5% d'acide péracétique.

Les micro gouttelettes de biocides sont générées à température ambiante à l'aide du générateur à ultrasons 20 à buse acoustique 33 de manière connue en soi. Ces micro gouttelettes, injectées dans l'air porteur grâce au générateur 10, circulent donc avec cet air porteur dans l'enceinte 18, dont les surfaces sont ainsi désinfectées.

La durée de l'injection des micro gouttelettes de biocide liquide dans le gaz porteur porté à la température de transport est dépendante du volume de l'enceinte 18 et de la puissance du générateur 20 de biocide mis en oeuvre. Par exemple, cette injection peut durer entre 20 et 45 minutes. Une fois la quantité souhaitée de biocide injectée dans le gaz porteur, on fait circuler le gaz porteur chargé du biocide dans l'enceinte pendant 30 à 60 minutes.

Généralement, on régule la concentration du biocide dans le gaz porteur pendant cette période de 30 à 60 minutes. Au cours de cette étape, on régule la température de l'air de façon que la différence entre la température de l'enceinte et de la température à laquelle se condense le biocide reste sensiblement comprise entre 5 et 10°C. Ces régulations sont habituellement gérées par un logiciel conçu à cet effet, apte à déterminer la quantité de biocide à pulvériser dans l'air et à déterminer la température de l'air.

Enfin, le procédé comporte, une fois le traitement terminé, une étape 130 de rinçage de l'enceinte en y faisant circuler de l'air dépourvu de biocide, et par exemple ce pendant 120 à 300 minutes, en brassant l'air à grande vitesse, par exemple à vitesse maximale, grâce aux moyens 16 de circulation d'air de la centrale de traitement d'air. On notera que, lorsque le biocide utilisé est du peroxyde d'hydrogène, ce peroxyde d'hydrogène finit, lors de cette étape, par se détériorer en eau H20 et en dioxyde 02, ne laissant ainsi dans l'enceinte que ces éléments non toxiques une fois le procédé terminé.

De préférence, l'air brassé au cours de cette étape est chauffé entre 30 et 40°C par les moyens de chauffage 12. En effet, l'air chaud favorise l'évaporation du biocide micro condensé sur les parois de l'enceinte 18 et donc favorise le rinçage de cette enceinte 18.

On notera que le générateur 20 de micro gouttelettes comporte de préférence des moyens d'alimentation et de vidange alternées en biocide et en eau pour les phases d'injection de micro gouttelettes de biocide et de nettoyage de ce générateur 20.

De préférence, le système d'aéraulique comporte une boucle aéraulique 35 agencée en parallèle du générateur 20, de façon que l'air circule par cette boucle aéraulique 35 en phase de rinçage 130 et par le générateur 20 durant les autres phases. Une telle boucle aéraulique 35 comporte des matériaux catalyseurs destinés à catalyser le peroxyde d'hydrogène pendant la phase de rinçage 130, par exemple des matériaux plaqués au platine.

En variante, il est possible de catalyser le biocide en pulvérisant un produit catalysant chimique ou organique.

En effet, en variante, les micro gouttelettes de biocide pourraient être aspirés dans l'enceinte plutôt que soufflées. En outre, les micro gouttelettes pourraient être injectées dans l'air indifféremment en amont ou en aval des moyens de climatisation de la centrale de traitement d'air.

## Revendications

1. Procédé de désinfection de surface par voie aérienne, par diffusion de biocide liquide, du type dans lequel on envoie un biocide dans un gaz porteur circulant dans au moins une enceinte (18), **caractérisé en ce qu'**il comporte les étapes suivantes :
- abaissement (100) de la température du gaz circulant dans l'enceinte (18) jusqu'à une température de refroidissement d'au plus 22°C, de préférence au plus 19°C, jusqu'à ce que les parois de l'enceinte (18) aient atteint cette même température,
- réchauffement (110) du gaz circulant dans l'enceinte (18) jusqu'à une température de transport d'au moins 35°C, de préférence au moins 40°C.
- génération (120) de micro gouttelettes de biocide, de granulométrie de 2 micromètres, et injection de ces micro gouttelettes dans le gaz porteur circulant dans l'enceinte (18) à la température de transport,
dans lequel on utilise un biocide liquide choisi parmi un composé à base de peroxyde d'hydrogène (H2O2), par exemple une solution de peroxyde d'hydrogène concentré à 30% dans de l'eau, de l'acide péracétique, ou un mélange de peroxyde d'hydrogène et d'acide péracétique, comprenant de préférence entre 2% et 5% d'acide péracétique.

2. Procédé selon la revendication 1, dans lequel les micro gouttelettes sont générées à température ambiante.

3. Procédé selon la revendication 1 ou 2, dans lequel la génération de micro gouttelettes est réalisée à l'aide d'un générateur à ultrasons (20) à buse acoustique (33).

4. Procédé selon l'une quelconque des revendications 1 à 3, dans lequel, lors de l'étape (100) consistant à abaisser la température du gaz porteur circulant dans l'enceinte, on fixe également l'hygrométrie relative dans ce gaz porteur en dessous de 60%, voire si possible en dessous de 50%, l'idéal étant 40%.

5. Procédé selon l'une quelconque des revendications précédentes, dans lequel le gaz porteur est de l'air.

6. Procédé selon l'une quelconque des revendications précédentes, dans lequel, une fois la température du gaz porteur égale à la température de refroidissement, on stabilise la température des parois de l'enceinte en maintenant la circulation du gaz porteur pendant une durée allant de 20 à 50 minutes.

7. Procédé selon l'une quelconque des revendications précédentes, dans lequel l'injection des micro gouttelettes de biocide liquide dans le gaz porteur porté à la température de transport dure entre 20 et 45 minutes.

8. Procédé selon l'une quelconque des revendications précédentes, dans lequel, une fois la quantité souhaitée de biocide injectée dans le gaz porteur, on fait circuler le gaz porteur chargé du biocide dans l'enceinte pendant 30 à 60 minutes.

9. Procédé selon la revendication précédente, dans lequel on régule la concentration du biocide dans le gaz porteur pendant la période de 30 à 60 minutes.

10. Procédé selon l'une quelconque des revendications précédentes, dans lequel, une fois le traitement terminé, on rince (130) l'enceinte (18) en y faisant circuler un gaz dépourvu de biocide, et ce pendant 120 à 300 minutes, en brassant le gaz à vitesse maximale, et de préférence en chauffant le gaz circulant dans l'enceinte entre 30 et 40°C.

## Claims

1. Method for disinfecting a surface by aerial way, by diffusing a biocidal liquid, the method being the type in which a biocide is sent into a carrier gas flowing in at least one enclosure (18), the method being **characterized in that** it comprises the following steps:
lowering (100) the temperature of the gas flowing in the enclosure (18) to a cooling temperature of no more than 22°C, preferably of no more than 19°C, until the walls of the enclosure (18) have reached the same temperature;
• heating (110) the gas flowing in the enclosure (18) up to a transport temperature of at least 35°C, preferably of at least 40°C; and
• generating (120) micro droplets of biocide, having a drop size of 2 micrometers, and injecting the micro droplets into the carrier gas flowing in the enclosure (18) at the transport temperature;
wherein a liquid biocide is used that is selected from a compound based on hydrogen peroxide (H₂O₂), e.g. a solution of hydrogen peroxide at a concentration of 30% in water, on peracetic acid, or on a mixture of hydrogen peroxide and of peracetic acid, preferably comprising in the range 2% to 5% of peracetic acid.

2. Method according to claim 1, wherein the micro droplets are generated at ambient temperature.

3. Method according to claim 1 or claim 2, wherein the micro droplets are generated using an ultrasound generator (20) having an acoustic nozzle (33).

4. Method according to any one of claims 1 to 3, wherein, during the step (100) consisting in lowering the temperature of the carrier gas flowing in the enclosure, the relative humidity of said carrier gas is also set to below 60%, and if possible to below 50%, the ideal being 40%.

5. Method according to any preceding claim, wherein the carrier gas is air.

6. Method according to any preceding claim, wherein, once the temperature of the carrier gas is equal to the cooling temperature, the temperature of the walls of the enclosure is stabilized by maintaining the flow of carrier gas for a duration lying in the range 20 minutes (min) to 50 min.

7. Method according to any preceding claim, wherein micro droplets of liquid biocide are injected into the carrier gas raised to the transport temperature for a duration lying in the range 20 min to 45 min.

8. Method according to any preceding claim, wherein, once the desired quantity of biocide has been injected into the carrier gas, the carrier gas loaded with biocide is caused to circulate inside the enclosure for a period in the range 30 min to 60 min.

9. Method according to the preceding claim, wherein the concentration of the biocide in the carrier gas is regulated during the period in the range 30 min to 60 min.

10. Method according to any preceding claim, wherein, once the treatment has finished, the enclosure (18) is rinsed (130) by causing a biocide-free gas to flow therein, and doing so for a period in the range 120 min to 300 min, while driving the gas flow at maximum speed, and preferably while heating the gas flowing in the enclosure to a temperature in the range 30°C to 40°C.

## Patentansprüche

1. Verfahren zur Flächendesinfektion über die Luft, durch Verteilung flüssigen Biozids, wobei ein Biozid in ein Trägergas geleitet wird, das in zumindest einem Raum (18) zirkuliert, **dadurch gekennzeichnet, dass** es die folgenden Schritte umfasst:
- Senkung (100) der Temperatur des im Raum (18) zirkulierenden Gases bis auf eine Kühltemperatur von höchstens 22 °C, vorzugsweise höchstens 19 °C, bis die Wände des Raums (18) eben diese Temperatur erreicht haben,
- Erhitzung (110) des im Raum (18) zirkulierenden Gases bis auf eine Transporttemperatur von wenigstens 35 °C, bevorzugt wenigstens 40 °C,
- Erzeugung (120) von Biozidmikrotröpfchen, mit einer granulometrischen Größe von 2 Mikrometern, und Einspritzung dieser Mikrotröpfchen in das Trägergas, das im Raum (18) mit der Transporttemperatur zirkuliert,
wobei ein flüssiges Biozid verwendet wird, gewählt aus einer Verbindung auf Wasserstoffperoxid-(H₂O₂)-Basis, beispielsweise einer 30%-igen Lösung von Wasserstoffperoxid in Wasser, Peressigsäure oder einer Mischung aus Wasserstoffperoxid und Peressigsäure, die vorzugsweise zwischen 2% und 5% Peressigsäure umfasst.

2. Verfahren nach Anspruch 1, wobei die Mikrotröpfchen bei Umgebungstemperatur erzeugt werden.

3. Verfahren nach Anspruch 1 oder 2, wobei die Erzeugung von Mikrotröpfchen mit Hilfe eines Ultraschallgenerators (20) mit Schalldüse (33) durchgeführt wird.

4. Verfahren nach einem der Ansprüche 1 bis 3, wobei, während des Schritts (100), der im Senken der Temperatur des im Raum zirkulierenden Trägergases besteht, weiterhin der relative Feuchtigkeitsgehalt in diesem Trägergas unterhalb von 60%, falls möglich sogar unterhalb von 50%, idealerweise auf 40% fixiert wird.

5. Verfahren nach einem der vorstehenden Ansprüche, wobei das Trägergas Luft ist.

6. Verfahren nach einem der vorstehenden Ansprüche, wobei, sobald die Temperatur des Trägergases gleich der Kühltemperatur ist, die Temperatur der Wände des Raums stabilisiert wird, indem die Zirkulation des Trägergases während einer Dauer von 20 bis 50 Minuten aufrechterhalten wird.

7. Verfahren nach einem der vorstehenden Ansprüche, wobei die Einspritzung der Mikrotröpfchen flüssigen Biozids ins auf die Transporttemperatur gebrachte Trägergas zwischen 20 und 45 Minuten dauert.

8. Verfahren nach einem der vorstehenden Ansprüche, wobei, sobald die gewünschte Biozidmenge ins Trägergas eingespritzt ist, das mit dem Biozid beladene Trägergas im Raum während 30 bis 60 Minuten in Zirkulation gehalten wird.

9. Verfahren nach dem vorstehenden Anspruch, wobei die Konzentration des Biozids im Trägergas während des Zeitraums von 30 bis 60 Minuten reguliert wird.

10. Verfahren nach einem der vorstehenden Ansprüche, wobei, sobald die Behandlung beendet ist, der Raum (18) gespült wird (130), wobei darin ein Gas ohne Biozid in Zirkulation gehalten wird, und dies während 120 bis 300 Minuten, wobei das Gas mit Höchstgeschwindigkeit umgewälzt wird, und wobei vorzugsweise das im Raum zirkulierende Gas auf 30 bis 40 °C erhitzt wird.
